# EUROPEAN PATENT SPECIFICATION

(11) **EP 2 653 556 B1**
(45) Date of publication and mention of the grant of the patent: **02.09.2015**
(21) Application number: 12165009.7
(22) Date of filing: 20.04.2012
(51) Int. Cl.: C12Q 1/68, G01N 33/50

(54) **CDR sequence-specific enrichment of live immune cells**
CDR-sequenzspezifische Anreicherung lebender Immunzellen
Enrichissement spécifique à la séquence CDR des cellules immunitaires vivantes

(43) Date of publication of application: 23.10.2013
(73) Proprietor: Alacris Theranostics GmbH, 14195 Berlin (DE)
(72) Inventor: Glökler, Jörn, 10783 Berlin (DE); Warnatz, Hans-Jörg, 10245 Berlin (DE)
(74) Representative: CH Kilger Anwaltspartnerschaft mbB

(56) References cited:
- WO-A2-2005/079462
- WO-A2-2007/116408
- FRANK W KING ET AL: "High-throughput tracking of pluripotent human embryonic stem cells with dual fluorescence resonance energy transfer molecular beacons.", STEM CELLS DEV., vol. 20, no. 3, 14 September 2010 (2010-09-14), pages 475-484, XP55036631,

### SEQUENCE LISTING

<110> Alacris Theranostics GmbH
<120> CDR sequence-specific enrichment of live immune cells
<130> B106-0001EP1
<160> 34
<170> PatentIn version 3.5
<210> 1
   <211> 318
   <212> DNA
   <213> Artificial Sequence
<220>
   <223> 5E4_VL sequence of IgM hybridoma 5E4 VL region
<400> 1
   agcccgtttt atttccaact ttgtccccga gccgaacgtg aatggaacat gtgaaccttg      60
   aaagcagtaa taaactccca gatcctcagc ctccactctg ctgatcttga gtgtgaaatc     120
   tgtccctgat ccactgccac tgaacctgtc tgggacccca gaaaatcggt tggaaacttt     180
   gtagatcagg agctttggag actggcctgg tttctgcagg taccattcta aataggtgtc     240
   tccattacta tgtacaatgc tctgactaga tctgcaagag atggaggctt gatctccaag     300
   actgacagtc agggagag                                                   318
<210> 2
   <211> 340
   <212> DNA
   <213> Artificial Sequence
<220>
   <223> 5E4_VH sequence of IgM hybridoma 5E4 VH region
<400> 2
   tgaggagact gtgagagtgg tgccttggcc ccagtagtca ccgctactca tagtaccctc      60
   cctggtacag taataaatgc cagtgtcttc aggtcttaag ttgttcattt gcaggtagac     120
   actacttttg gaatcatctc ttgagatggt gaacctccct ttcacagact ccgcataatg     180
   tgttgcataa tttccagatt tcattctgat ttcagcaacc cactcaagtc ccttctctgg     240
   agactggcgg acccagttca tccagtagtt actgaaagtg aatccagagg caacacagga     300
   gagtttcatg gatcctccag gttgcaccaa gcctcctcca                           340
<210> 3
   <211> 306
   <212> DNA
   <213> Artificial Sequence
<220>
   <223> KT13_VL sequence of IgM hybridoma KT13 VL region
<400> 3
   agcccgtttc agctccagct tggtcccagc accgaacgtg agtgggtaac cactgtactg 60
   ctggcagtaa taagtggcag catcttcagc ctccacactg ctgattgtga gagagtaaga 120
   ggtcccagac ccactgccac tgaagcgagc agggactcca gaagccaagt tggatgtgct 180
   ataaatccag agtttggggg aggcacctga cttctgctgg taccagtgca agtaactgga 240
   acttacactt gagctggccc tgcaggtcat ggtgaccttt tcccctggag atgcagacat 300
   gattgc 306
<210> 4
   <211> 334
   <212> DNA
   <213> Artificial Sequence
<220>
   <223> KT13_VH sequence of IgM hybridoma KT13 VH region
<400> 4
   tgaggagacg gtgactgagg ttccttgacc ccagtagtcc aaaacatagg ggtaaacgga 60
   tcttgcacag taatagactg cagagtcctc agatgtcagg ctgctcagtt gcatgtaggc 120
   tgtgctggag gatttgtctg cagtcaatgt ggccttgtcc ttgaacttct gattgtactc 180
   aatataagca gtgctaggat taatgtatcc aatccattcc agaccctgtc caggcctctg 240
   ttttacccag tgcatccagt aggtactaaa ggtgtagcca gaagccttgc aggacatctt 300
   cactgaggcc ccaggttttg ccagttcaac ccca 334
<210> 5
   <211> 303
   <212> DNA
   <213> Artificial Sequence
<220>
   <223> KT22_VL sequence of IgM hybridoma KT22 VL region
<400> 5
   agcccgtttt atttccagct tggtcccccc tccgaacgtg tacggaaact gagcatactg 60
   tacacagtaa tagtctgcaa aatcttcaga ttccaggctg ctgatggtga gagaataatc 120
   tgctccagat ccactgccac tgaaccttga tggaactcca tcttccaagt tggttccatg 180
   atagatcagg cccttaaatg atttccctgg tttctgctgc aaccacccta tattactgct 240
   aatgccctga cttgcatggc aagtgatgct gactgtgtct cccagagata cagacatgga 300
   gga 303
<210> 6
   <211> 343
   <212> DNA
   <213> Artificial Sequence
<220>
   <223> KT22_VH sequence of IgM hybridoma KT22 VH region
<400> 6
   tgaggagacg gtgaccgtgg tccctgcgcc ccagacatcg aagtaccaca ggtagccgta    60
   gtaatatcgg cctcttgcac agtaatacat ggccgtgtcc tcagacctca gactgctcat   120
   ttgcaggtac aggatgttcc tggcattatc tctggagatg gtgaatcggc ccttcacact   180
   gtctggatag taggtgctac caccactact aatggatgcg acccactcca gcctcttctc   240
   tggagtctgg cgaacccaag acatggcata gctactgaaa gtgaatccag aggctgcaca   300
   ggagagtttc agggaccctc caggcttcac taagcctccc cca                     343
<210> 7
   <211> 23
   <212> RNA
   <213> Artificial Sequence
<220>
   <223> KT22VLDonor (23 nt, 2'-O-Me RNA); probe
<400> 7
   gccaugcaag ucagggcauu ggc 23
<210> 8
   <211> 24
   <212> RNA
   <213> Artificial Sequence
<220>
   <223> KT22VLAcceptor (24 nt, 2'-O-Me RNA); probe
<400> 8
   gaugcgggag acacagucag cauc 24
<210> 9
   <211> 25
   <212> RNA
   <213> Artificial Sequence
<220>
   <223> KT22VHDonor (25 nt, 2'-O-Me RNA); probe
<400> 9
   gcaccuacua uccagacagu ggugc 25
<210> 10
   <211> 25
   <212> RNA
   <213> Artificial Sequence
<220>
   <223> KT22VHAcceptor (25 nt, 2'-O-Me RNA); probe
<400> 10
   accacucgca uccauuagua guggu 25
<210> 11
   <211> 23
   <212> DNA
   <213> Artificial Sequence
<220>
   <223> 5E4_VL_fwd forward primer for 5E4 VL_region
<400> 11
   cccagaaaat cggttggaaa ctt 23
<210> 12
   <211> 24
   <212> DNA
   <213> Artificial Sequence
<220>
   <223> 5E4_VL_rev reverse primer for 5E4 VL_region
<400> 12
   tcaagcctcc atctcttgca gatc 24
<210> 13
   <211> 22
   <212> DNA
   <213> Artificial Sequence
<220>
   <223> KT13_VL_fwd forward primer for KT13 VL region
<400> 13
   ccagaagcca agttggatgt gc 22
<210> 14
   <211> 22
   <212> DNA
   <213> Artificial Sequence
<220>
   <223> KT13_VL_rev reverse primer for KT13 VL region
<400> 14
   aaaggtcacc atgacctgca gg 22
<210> 15
   <211> 24
   <212> DNA
   <213> Artificial Sequence
<220>
   <223> KT22_VL_fwd forward primer for KT22 VL region
<400> 15
   ctccatcttc caagttggtt ccat 24
<210> 16
   <211> 22
   <212> DNA
   <213> Artificial Sequence
<220>
   <223> KT22_VL_rev reverse primer for KT22 VL region
<400> 16
   cagtcagcat cacttgccat gc 22
<210> 17
   <211> 23
   <212> DNA
   <213> Artificial Sequence
<220>
   <223> 5E4_VH_fwd forward primer for 5E4 VH region
<400> 17
   agatggtgaa cctccctttc aca 23
<210> 18
   <211> 23
   <212> DNA
   <213> Artificial Sequence
<220>
   <223> 5E4_VH_rev reverse primer for 5E4 VH region
<400> 18
   gccagtctcc agagaaggga ctt 23
<210> 19
   <211> 22
   <212> DNA
   <213> Artificial Sequence
<220>
   <223> KT13_VH_fwd forward primer for KT13 VH region
<400> 19
   tcaatgtggc cttgtccttg aa 22
<210> 20
   <211> 22
   <212> DNA
   <213> Artificial Sequence
<220>
   <223> KT13_VH_rev reverse primer for KT13 VH region
<400> 20
   ctgggtaaaa cagaggcctg ga 22
<210> 21
   <211> 22
   <212> DNA
   <213> Artificial Sequence
<220>
   <223> KT22_VH_fwd forward primer for KT22 VH region
<400> 21
   gaatcggccc ttcacactgt ct 22
<210> 22
   <211> 22
   <212> DNA
   <213> Artificial Sequence
<220>
   <223> KT22_VH_rev reverse primer for KT22 VH region
<400> 22
   atgtcttggg ttcgccagac tc 22
<210> 23
   <211> 22
   <212> DNA
   <213> Artificial Sequence
<220>
   <223> Hspa5_fwd control forward primer for mouse Hspa5
<400> 23
   aaaccaagac atttgcccca ga 22
<210> 24
   <211> 22
   <212> DNA
   <213> Artificial Sequence
<220>
   <223> Hspa5_rev control reverse primer for mouse Hspa5
<400> 24
   cagcatcttt ggttgcttgt cg 22
<210> 25
   <211> 24
   <212> RNA
   <213> Artificial Sequence
<220>
   <223> Jurkat TCR alpha probe; Donor (24 nt, 2'-O-Me RNA)
<400> 25
   gagaucacuc acagcacaga ucuc 24
<210> 26
   <211> 24
   <212> RNA
   <213> Artificial Sequence
<220>
   <223> Jurkat TCR alpha probe; Acceptor (24 nt, 2'-O-Me RNA)
<400> 26
   cgaacuugga agcacugcug uucg 24
<210> 27
   <211> 23
   <212> RNA
   <213> Artificial Sequence
<220>
   <223> Jurkat TCR beta probe; Donor (23 nt, 2'-O-Me RNA)
<400> 27
   caggucgaga aacugcuggc cug 23
<210> 28
   <211> 26
   <212> RNA
   <213> Artificial Sequence
<220>
   <223> Jurkat TCR beta probe; Acceptor (26 nt, 2'-O-Me RNA)
<400> 28
   gcuaacaagg uguagccaua guuagc 26
<210> 29
   <211> 22
   <212> DNA
   <213> Artificial Sequence
<220>
   <223> Jurkat_TCRA_fwd forward primer for Jurkat TCR alpha sequence
<400> 29
   gaaacctcct tccacctgac ga 22
<210> 30
   <211> 22
   <212> DNA
   <213> Artificial Sequence
<220>
   <223> Jurkat_TCRA_rev reverse primer for Jurkat TCR alpha sequence
<400> 30
   tatttggccg gatgctgagt ct 22
<210> 31
   <211> 22
   <212> DNA
   <213> Artificial Sequence
<220>
   <223> Jurkat_TCRB_fwd forward primer for Jurkat TCR beta sequence
<400> 31
   tgaagatcca gccctcagaa cc 22
<210> 32
   <211> 20
   <212> DNA
   <213> Artificial Sequence
<220>
   <223> Jurkat_TCRB_rev reverse primer for Jurkat TCR beta sequence
<400> 32
   ctcgggtggg aacaccttgt 20
<210> 33
   <211> 24
   <212> DNA
   <213> Artificial Sequence
<220>
   <223> HSPA5_fwd control forward primer for human HSPA5
<400> 33
   cacagtggtg cctaccaaga agtc 24
<210> 34
   <211> 25
   <212> DNA
   <213> Artificial Sequence
<220>
   <223> HSPA5_rev control reverse primer for human HSPA5
<400> 34
   caggaggaat tccagtcaga tcaaa 25

## Description

### INTRODUCTION

The adaptive immune system of vertebrates is antigen-specific due to a large repertoire of both B lymphocytes producing antibodies and T cells presenting antigen-specific receptors.

The capacity of lymphocytes to generate a heterogeneous repertoire of antigen binding proteins is the basis of their ability to recognize a broad range of specific antigens. The antigen receptors are encoded by families of variable (V), diversity (D) and joining (J) segments and constant (C) domains that undergo rearrangements exclusively in lymphoid cells. The antigen binding site of the B cell antigen receptor is created by the V domains of paired heavy (H) and light (L) chains. Each variable domain contains three intervals of highly variable sequence, the complementary determining regions (CDRs), which are separated from each other by four intervals of conserved sequences, the frameworks (FRs). The variability of the CDRs, which are responsible for antigen specificity, constitutes the diversity of the human immune repertoire.

In the immune repertoire, the CDRs 1 and 2 are exclusively encoded by germline V gene segment sequences. The light chain CDR3 (LCDR3) is created by joining of V_{L} and J_{L} gene segments and introduces a moderate amount of somatic diversity to the repertoire.

The third H chain CDR (HCDR3) is the most diverse component of the antigen binding pocket in antibodies and typically plays a critical role in defining the specificity of the antibody. Most HCDR3 intervals include a D gene segment that is typically flanked by runs of non-germline-encoded nucleotides (N regions). Nucleotides palindromic to the termini of the rearranging gene segments, termed P junctions, can also be added. Inclusion of a D gene segment, addition of N nucleotides, and gene segment extension by up to four sets of P junctions make HCDR3 the focus of somatic diversification of the preimmune repertoire (Shiokawa et al., J Immunol, 1999, 162:6060-6070).

T cells are produced in the thymus in a high number, each of which expresses a unique T cell receptor (TCR) capable of binding to a foreign peptide in the binding groove of a host major histocompatibility complex (MHC) molecule. The binding between TCR and antigen is of relatively low affinity and is degenerate: that is, many TCR recognize the same antigen and many antigens are recognized by the same TCR.

The TCR is a heterodimer being composed of two different protein chains. In 95% of T cells, this consists of an alpha (α) and beta (β) chain, whereas in 5% of T cells this consists of gamma (γ) and delta (δ) chains. Each chain is divided into a constant (C) and variable (V) region. The constant region has an end that is anchored in the cell membrane. The variable region faces outward and binds to the HLA molecule and the antigen it presents.

The structure of TCR is very similar to immunoglobulin Fab fragments. Each chain of the TCR is a member of the immunoglobulin superfamily and possesses one N-terminal immunoglobulin (Ig)-variable (V) domain, one Ig-constant (C) domain, a transmembrane/cell membrane-spanning region, and a short cytoplasmic tail at the C-terminal end. The variable domain of both the TCR α-chain and β-chain have three hypervariable or complementarity determining regions (CDRs), whereas the variable region of the β-chain has an additional area of hypervariability (HV4) that does not normally contact antigen and, therefore, is not considered a CDR. The residues are located in two regions of the TCR, at the interface of the α- and β-chains and in the β-chain framework region that is thought to be in proximity to the CD3 signal-transduction complex. CDR3 is the main CDR responsible for recognizing processed antigen, although CDR1 of the alpha chain has also been shown to interact with the N-terminal part of the antigenic peptide, whereas CDR1 of the β-chain interacts with the C-terminal part of the peptide. CDR2 is thought to recognize the MHC. CDR4 of the β-chain is not thought to participate in antigen recognition, but has been shown to interact with superantigens.

The process for TCR formation is similar to those described for antibodies. The TCR alpha chain is generated by VJ recombination, whereas the beta chain is generated by V(D)J recombination which involves a somewhat random joining of gene segments to generate the complete TCR chain. Likewise, generation of the TCR gamma chain involves VJ recombination, whereas generation of the TCR delta chain occurs by V(D)J recombination. The intersection of these specific regions (V and J for the alpha or gamma chain; V, D, and J for the beta or delta chain) corresponds to the CDR3 region that is important for antigen-MHC recognition.

It is the unique combination of the segments at this region, along with palindromic and random N-and P-nucleotide additions, which accounts for the great diversity in specificity of the T cell receptor for processed antigen.

When the TCR engages with antigen and MHC, the T lymphocyte is activated through a series of biochemical events mediated by associated enzymes, co-receptors, specialized accessory molecules, and activated or released transcription factors. T cell-mediated immunity to infection is due to the proliferation and differentiation of rare clones in the preimmune repertoire that by chance express TCRs specific for peptide-MHC (pMHC) ligands derived from the microorganism (Jenkins et al., Annu Rev Immunol, 2010, 28:275-94).

By using next generation sequencing technologies, it is possible to characterize the immune repertoire on the basis of their nucleic acid sequence alone. Through comparison of clonal abundances in immunized individuals it is possible to delineate the sequences belonging to antigen-specific clones.

Since both antibodies and T cell receptors are generally multimeric proteins encoded by separate mRNAs, obtaining the right combination in great diversities is a difficult task to accomplish. The corresponding mRNAs can be found by either correlating the relative abundance (Reddy, S. T. et al. Monoclonal antibodies isolated without screening by analyzing the variable-gene repertoire of plasma cells. Nat. Biotechnol. 28, 965-969 (2010)), single-cell assembly PCR (Embleton et al., Nucleic Acids Res, 1992, 20: 3831-3837; Chapal et al., BioTechniques, 1997, 23:518-524), or sub-cellular assembly PCR in emulsion (Glökler, Warnatz, Lehrach, European Patent Application EP12154726 filed on 09 February 2012). In some cases antibodies can consist only of a heavy chain homodimer without an additional light chain. Camelids and cartilaginous fish such as sharks are known to possess a subpopulation of heavy-chain-like antibodies.

Assembled variable regions can be used to reconstruct either complete or fragments of antigen-specific immunoglobulins for either characterization or therapy. Especially TCRs can only be effective in therapy if they can be obtained as original complete T cells, preferentially as autologous transfusions. So far, antigen-specific T cells could only be identified and isolated based on incubation with labeled MHC/peptide complexes and subsequent fluorescence activated cell sorting (Dunbar et al., Curr. Biol., 1998, 8:413-416).

Aside from this, adoptive cell therapy (ACT), that employs less directed transfer of specific T cells, suffers from several problems (Grupp et al., Curr Top Microbiol Immunol, 2011, 344:149-172): Two subsets of memory cells, "effector memory" (TEM) and "central memory" (TCM), were originally identified on the basis of tissue homing molecules and effector function (Sallusto et al., Annu Rev Immunol, 2004, 22: 745-763). TEM can be found in the tumor, but are less effective probably by limited renewal and expansion capacity. Thus there is a need to identify the specific TCM that can be more efficient in treatment.

Until now only dead B cells have been analyzed by FACS without any sorting based on specific CDRs (Cao et al., J Clin Invest, 1995, 95:964-972).

Mouse monoclonal antibodies (mAbs) derived from immortalized B cells (hybridomas) have revolutionized molecular biology. However, therapeutic antibodies have to be humanized in order not to provoke any human anti-mouse response (HAMA). Such humanization is a cumbersome and complicated process that may also result in loss of specificity. In order to overcome these problems, transgenic mouse strains have been established with a largely humanized antibody repertoire. Still, it would be advantageous to directly generate human monoclonal antibodies from human individuals. In contrast to mouse, human B cells are much more difficult to establish as a stable immortalized cell line. Current approaches use Epstein-Barr virus (EBV) transformation with additional stimulations. Even with advances to increase the efficiency of transformation, these methods still suffer from several drawbacks. First, viral transformation causes cells lines to be unstable. Second, memory B cells were used to produce the cell lines. Since memory B cells represent the entire immunological history of the patient and not necessarily the infection or vaccination of interest, it is not guaranteed that all or most of these monoclonal antibodies will be relevant. This makes it necessary to screen many cell lines before a relevant clone is identified (Sullivan et al., F1000 Reports Biology, 2011, 3:17).

Pluripotent human embryonic stem cells have been isolated by FACS with dual-labeled molecular beacons specific to the Oct4 mRNA (King et al., Stem Cells Dev, 2011, 20:475-484) without affecting their viability or ability to differentiate. However, the separation was not based on clonality since the genetic background of somatic cells is supposed to be identical.

To date antibodies and TCRs are exclusively selected on the basis of their antigen specificity. This approach is expensive and time-consuming.

Hence, there is an unmet need to identify and enrich clonal cells, particularly immune cells, based on their characteristic and antigen-specific variable sequences alone.

### DESCRIPTION OF THE INVENTION

The present invention solves the problem in providing a method of clonally sorting and isolating living immune cells, which have a specific ribonucleic acid molecule in common, wherein the ribonucleic acid molecule is the transcriptional product of at least two different genomic regions, the method comprising the steps of:
a. providing a sample comprising said living immune cells;
b. providing at least one labeled probe set, each probe set comprising
   i. a first probe which is specific for a region of the ribonucleic acid stemming from a first genomic region and,
   ii. a second probe which is specific for a region of the ribonucleic acid stemming from a second genomic region and,
c. transfecting said cells with said probe set such that hybridization to said regions of the ribonucleic acid molecule may occur and if said hybridization occurs the probes lie adjacent to one another on said ribonucleic acid molecule;
d. detecting hybridization of said probe set to said nucleic acid regions; and
e. sorting the cells depending on how hybridization to said regions has occurred,
wherein the immune cells are hybridoma cells and/or B cells and
wherein at least two genomic regions encode for different complementary determining regions of the light chain or heavy chain of an antibody.

The invention is based on the finding that by using the nucleic acid sequence information of variable domains of a given immunoglobulin an antibody having the desired properties can be identified directly in a living cell without affecting the viability of said cell.

The present invention has the advantage that (a) the isolation of a corresponding heterodimer mRNA to a known sequence is possible, (b) live immune cells can be specifically isolated for downstream applications, (c) autologous B cells can be obtained for personalized immunotherapy, and (d) the isolation is fast and inexpensive and does not require any recombinant techniques or proteins. Moreover, dead cells may also be used for paired variable domain sequencing alone.

### DETAILED DESCRIPTION OF THE INVENTION

The present invention relates to a method of clonally sorting and isolating living cells, which have a specific ribonucleic acid (RNA) molecule in common, wherein the ribonucleic acid molecule is the transcriptional product of at least two different genomic regions, the method comprising the steps of:
a. providing a sample comprising said living cells;
b. providing at least one labeled probe set, each probe set comprising
   i. a first probe which is specific for a region of the ribonucleic acid stemming from a first genomic region and,
   ii. a second probe which is specific for a region of the ribonucleic acid stemming from a second genomic region and,
c. transfecting said cells with said probe set such that hybridization to said regions of the ribonucleic acid molecule may occur and if said hybridization occurs the probes lie adjacent to one another on said ribonucleic acid molecule;
d. detecting hybridization of said probe set to said nucleic acid regions; and
e. sorting the cells depending on how hybridization to said regions has occurred.
wherein the immune cells are hybridoma cells and/or B cells and wherein at least two genomic regions encode for different complementary determining regions of the light chain or heavy chain of an antibody.

The present invention allows for detecting, sorting and isolating cells having a RNA molecule in common which is the transcriptional product of at least two different genomic regions.

Herein, a genomic region is preferably a gene segment. The first gene segment and the second gene segment may preferably undergo recombination upon immunoglobulin formation resulting in a transcriptional product comprising the first and the second gene segment. Additional hypermutation may occur in the segments during affinity maturation. Preferably, the parts stemming from the first and the second gene segment lie in the transcriptional product adjacent to each other. If FRET is used for detecting and sorting the cells, the distance between the two genomic regions is ca. 4 nucleotides.

A genomic region may be selected from the group of V, D, J segments and C domains. Virtually all combinations are possible. For example, the first genomic region may be a V segment and the second genomic region a D segment, or the first genomic region may be a D segment and the second genomic region a J segment. Preferably, a genomic region is a V segment. More preferably, both genomic regions are V segments. It is most preferred that one genomic region encode for a first CDR, the other genomic region for a second CDR. For example, the first genomic region may encode for an antibody heavy (H) chain CDR1 and the second genomic region for a H chain CDR2, or the first genomic region encode for a H chain CDR2 and the second genomic region for a H chain CDR3.

However, a genomic region is not limited to genes or gene segments. A genomic region may also be an open reading frame (ORF). A genomic region may also include a mutation such as a single nucleotide polymorphism (SNP).

The invention makes use of probes. A probe is an oligonucleotide, nucleic acid or a fragment thereof, which is substantially complementary to a specific nucleic acid sequence. It is to be noted that probes need to fold correctly preferentially at 37°C (depending on the cell type and on whether live or dead cells are used) in order to specifically hybridize to cognate mRNAs in vivo (inside the target cell). The present method is not restricted to particular probes. Instead, various probes and assays may be used, including quenching-type binary probes, FRET-type binary probes, quenching-type molecular beacon probes and FRET-type molecular beacon probes. Hence, the step of detecting hybridization is preferably based on quenching or FRET.

The difference between quenching- and FRET-type probes is the mechanism by which energy is transferred from one label to the other. (Static) Quenching occurs when the donor and acceptor molecules are in the ground state. In this case, the acceptor (i.e. quencher) absorbs excitation energy from the donor and dissipates the energy as heat. In FRET, energy transfer occurs while the donor is in the excited state. Thus, a FRET acceptor re-emits much of this energy as light. The occurrence of FRET depends upon such conditions as the distance between the donor and acceptor fluorophores (typically 10-100 Å; Haugland et al. 1969. Proc. Natl Acad. Sci. USA 63:23-30.), the spectral overlap between the donor emission and acceptor absorption spectra, and the orientation of transition moments of both fluorophores. For the most frequently used fluorescein-rhodamine pair, the maximum transfer efficiency is generally obtained when the donor and acceptor are separated by four or five intervening nucleotides. For details and peculiarities see Cardullo et al. 1988. Proc Natl Acad Sci U S A. 85(23):8790-4.. At shorter distances, the contribution of non-FRET-based contact quenching significantly increases.

Binary probes utilize two probes hybridizing to adjacent DNA sequences. The probes may be end-labeled with chromophores so that one end is labeled at the 3' end and the other at the 5' end. An improvement with respect to the fluorescence intensity may be achieved by inserting two identical fluorophores close to each other into the donor probe.

Molecular beacon probes are hairpin shaped molecules with an internally quenched fluorophore whose fluorescence is restored when they bind to a target nucleic acid sequence. The stem is formed by the terminal 5-8 bases, while the central single-stranded portion assumes a loop configuration. It can be between 15 and 35 nucleotides long. The double-stranded stem carries the donor and acceptor dyes. The loop fragment can hybridize to the target nucleic acid sequence. When the probe is not hybridized to the target, the donor and acceptor are brought in close proximity by the stem formation, resulting in the FRET-based quenching of the donor. However, in the presence of the target sequence, the probe forms longer and stronger hybrids with the target than by forming the stem. The FRET transfer is disrupted, thus resulting in the appearance of the donor fluorescence.

To increase sensitivity and signal-to-background ratio a third fluorophore may be incorporated into the hairpin structure (e.g. FAM, TAMRA, and Cy5). FAM as the primary donor may be attached to one arm of the molecular beacon, whereas the primary energy acceptor/secondary donor (TAMRA) and the secondary acceptor (Cy5) may be located at the other terminus of the molecular beacon. Double-stranded probes formed by two complementary oligonucleotides may be used in the inventive method as well. In such assay one of the oligonucleotides is usually 5' end-labeled with the donor fluorophore, and the other has a 3' acceptor. The rationale of the assay is to detect the target sequence by competitive hybridization of one of the oligonucleotides forming the probe duplex. When the initial probe duplex is disrupted due to the hybridization of one of the oligonucleotides to the target sequence, the quenching of the donor is stopped and its fluorescence becomes observable.

A probe labeled with only an acceptor fluorophore may also be used. It works in combination with a low molecular weight donor dye, which intercalates into the target DNA. When the probe hybridizes to the target sequence, the energy transfer occurs from the intercalator to the acceptor, increasing its fluorescence. An example of this assay uses the intercalating dye acridine orange serving as a donor and rhodamine at the 5' end of the oligonucleotide probe acting as an acceptor.

In a preferred embodiment the probe is selected from the group of LightCycler probe (Roche), the TaqMan probe (Life Technologies), a Molecular Beacon probe, a Scorpion primer, a Sunrise primer, a LUX primer and an Amplifluor primer. Molecular beacon probes are most preferred in the context of the present invention.

The probes are preferably designed such that, if both genomic regions are present in the transcriptional product, they lie adjacent to one another. This means that the presence of both genomic regions may be detected by an interaction of the probes (or the labels of the probes). This interaction, for example, can be based on static quenching or FRET (see above). The terms "reporter" and "quencher" are conventionally used for quenching-type probes. However, for the sake of simplicity, these terms are herein used also in the context of FRET-type probes. In this case, a reporter may denote a donor and a quencher may denote an acceptor.

The probes used herein are labeled. Herein, a "label" is a moiety that is bound covalently or non-covalently to a probe or primer where it can give rise to a signal which may be detected by optical or other physical means. A label is preferably a fluorescent dye. In the context of the present invention, fluorescent dyes may for example be FAM (5- or 6-carboxyfluorescein), VIC, NED, fluorescein, fluorescein isothiocyanate (FITC), IRD-700/800, cyanine dyes, such as CY3, CY5, CY3.5, CY5.5, CY7, xanthen, 6-carboxy-2',4',7',4,7-hexachlorofluorescein (HEX), TET, 6-carboxy-4',5'-dichloro-2',7'-dimethodyfluorescein (JOE), N,N,N',N'-tetramethyl-6-carboxyrhodamine (TAMRA), 6-carboxy-X-rhodamine (ROX), 5-carboxyrhodamine-6G (R6G5), 6-carboxyrhodamine-6G (RG6), rhodamine, rhodamine green, rhodamine red, rhodamine 110, BODIPY dyes, such as BODIPY TMR, Oregon Green, coumarines such as umbelliferone, benzimides, such as Hoechst 33258; phenanthridines, such as Texas Red, Yakima Yellow, Alexa Fluor, PET, ethidium bromide, acridinium dyes, carbazol dyes, phenoxazine dyes, porphyrine dyes, polymethin dyes, and the like. The labeling of the probes must be such that each probe set can be distinguished from the other probe set since the labeling ultimately corresponds to the sequence on the template nucleic acid. This means that in one embodiment, the number of different labels used is proportional to the number of potential sequences to be read out on the transcriptional product. Whether the probes are double-labeled or single-labeled depends on the probe assay used in the inventive method (see above). In a preferred embodiment, the reporter is selected from the group of fluorescein dyes and the quencher is selected from the group of rhodamine dyes.

A probe set may comprise single-labeled probes or dual-labeled probe(s). There are a variety of different combinations possible, where even dual-labeled and single-labeled probe(s) are used together in a single probe set. Most importantly, a single probe set specifically hybridizes to an RNA molecule only if it stems from two different genomic regions. Accordingly, a single probe set allows for detecting RNA stemming from two different genomic regions.

A probe set may consist of only one probe, i.e. a molecular beacon probe. If only one probe is used, the probe may hybridize to a joint region of the RNA molecule where the segments stemming from the first and the second genomic region are recombined (joined together).

A probe set can also consist of 2 probes, i.e. a pair of probes. In this case, one probe may hybridize to the first genomic region, the other probe to the second genomic region. In a preferred embodiment, both probes may hybridize in the proximity of the joint region, wherein one probe may hybridize somewhat more upstream and the other probe somewhat more downstream of the joint region. It is preferred that the probes hybridize in close proximity to each other such that an interaction (for example FRET) occurs when both probes hybridize to the RNA. Most preferably, a probe set consists of two dual-labeled molecular beacon probes. For example, 6FAM may be used as the donor fluorophore and 5ROX as the acceptor molecule. BHQ1 may be used to quench 6FAM in the donor probe and BHQ2 to quench 5ROX in the acceptor probe when in solution as short hairpins. When hybridized to its target FRET shall occur between the donor and the acceptor molecule (see page 477 and 478 of King et al. for details).

A probe set preferably comprises 2 probes, but may also comprise 3, 4, 5 or 6 probes. Most importantly, the probes of the probe set are to be selected that they can be detected, if they specifically hybridize to the RNA molecule having the desired sequence properties, i.e. only if the RNA is the transcriptional product of at least two different pre-selected genomic regions.

In one embodiment, the probe set shall allow for differentiating between those cells that have the transcriptional product in common and those cells that do not bear the transcriptional product. In another embodiment, the probe set shall also allow for differentiating between those cells whose transcriptional product stems from only one pre-selected genomic region but lacks the other genomic region. This can be easily achieved by the use of different labels.

It is often desired to enhance the signal intensity, which can be achieved by using more than one probe, e.g. 2, 3, 4, or 5 probes, for the detection of the RNA part stemming from a single genomic region. Alternatively or additionally, the number of labels of a single probe may be increased (see above).

In the following, preferred probes sets are composed as exemplified:
(a) the first probe is a single-labeled probe and the second probe is a single-labeled probe, and one label consists of a reporter molecule and the other label consists of a quencher molecule.
(b) the first probe is a dual-labeled probe and the second probe is a dual-labeled probe, and each dual-label consists of a reporter molecule and a quencher molecule.
(c) the first probe is a dual-labeled probe and the second probe is a single-labeled probe, and the single-label consists of a quencher molecule.

In the inventive method even more than one probe set can be applied. This is of great advantage for the detection, identification or sorting of cells having a RNA molecule in common which is the transcriptional product stemming from more than two genomic regions. For example, 2, 3, 4, 5, 6, and so on, probe sets may allow for the detection of an RNA molecule stemming from 4, 6, 8, 10, 12 and so on, genomic regions, respectively. In certain cases, e.g. where three genomic regions are recombined consecutively, two probe sets can also be used for the detection of an RNA molecule stemming from three genomic regions. Accordingly, another embodiment of the invention makes use of a second probe set, wherein the second probe set comprises a third probe which is specific for a region of the ribonucleic acid stemming from a third genomic region and a fourth probe which is specific for a region of the ribonucleic acid stemming from a fourth genomic region.

In the context of the invention the RNA molecule may be mRNA or pre-mRNA; mRNA is preferred. Herein, a sample includes blood, serum, plasma, cerebrospinal fluid, skin, tumor tissue, lymphoid tissues, organs (spleen), bone marrow, urine, saliva, sputum, and pleural effusions. The sample preferably stems from a vertebrate, such as a human. The sample does not necessarily comprise live cells; the invention may be carried out on dead cells as well. In a preferred embodiment, the cells are viable immune cells. The immune cells are preferably hybridoma cells, and/or B cells

Transfection is the process of deliberately introducing nucleic acids into cells. Transfection of animal cells typically involves opening transient pores or "holes" in the cell membrane, to allow the uptake of material. Transfection can be carried out using calcium phosphate, by electroporation, by pore-forming proteins (streptolysin O), or by mixing a cationic lipid with the material to produce liposomes, which fuse with the cell membrane and deposit their cargo inside.

As used herein, hybridization means the pairing of complementary nucleic acid sequences. Hybridization will generally occur if there is at least 95% and preferably at least 97% sequence identity between the probe and the target sequence. The hybridization conditions obviously depend on whether viable or dead cells are used. In general, hybridization conditions are well known and exemplified in Sambrook *et al,* Molecular Cloning: A Laboratory Manual, Second Edition, Cold Spring Harbor, NY (1989), particularly chapter 11.

Sorting in the context of the present invention means grouping items with similar properties (e.g. cells having a specific RNA in common) together. In a preferred embodiment, the step of sorting the cells is based on fluorescence-activated cell sorting (FACS).

FACS is a method for sorting a heterogeneous mixture of biological cells into two or more containers, one cell at a time, based upon the specific light scattering and fluorescent characteristics of each cell. The cell suspension is entrained in the center of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell per droplet. Just before the stream breaks into droplets, the flow passes through a fluorescence measuring station where the fluorescent character of interest of each cell is measured. An electrical charging ring is placed just at the point where the stream breaks into droplets. A charge is placed on the ring based on the immediately prior fluorescence intensity measurement, and the opposite charge is trapped on the droplet as it breaks from the stream. The charged droplets then fall through an electrostatic deflection system that diverts droplets into containers based upon their charge. In some systems, the charge is applied directly to the stream, and the droplet breaking off retains charge of the same sign as the stream. The stream is then returned to neutral after the droplet breaks off.

Generally, the inventive method may be carried out as follows: First, one or more specific probe sets are designed. For this, known sequence information may be used. Alternatively, the identity of the genotype of desired cells may be determined by sequencing of DNA or RNA, e.g. by next-generation sequencing. We postulate that, since the chromosomal genes normally exist in only two copies, RNA derived from the genes exists in a higher copy number, resulting in signal amplification. Probes need to fold correctly preferentially at 37°C (depending on the cell type) in order to specifically hybridize to cognate mRNAs in vivo (inside the living target cells). The probes may be transferred into a cell population by standard protocols. Preferably, dual-labeled molecular beacon probes are used which provide a highly specific fluorescent signal depending on the desired genotype. The labeled cells may be then singled out either by micromanipulation or by FACSorting or other preferably droplet-based microfluidic techniques such as RainStorm^{™} without affecting the viability of the target cells. Furthermore, the mRNA may be extracted and the corresponding sequences of the multimeric proteins either identified through sequencing, or confirmed if already known.

The main difference to the prior art is the identification and separation of clonal cells differing in the genotype. Since the template gene exists in a low copy number in a high nucleic acid density inside the nucleus, RNA derived from the gene is an ideal target for specific probes. The RNA can be present in higher copy number preferentially outside the nucleus, thus providing signal amplification and lower background.

### FIGURE CAPTIONS

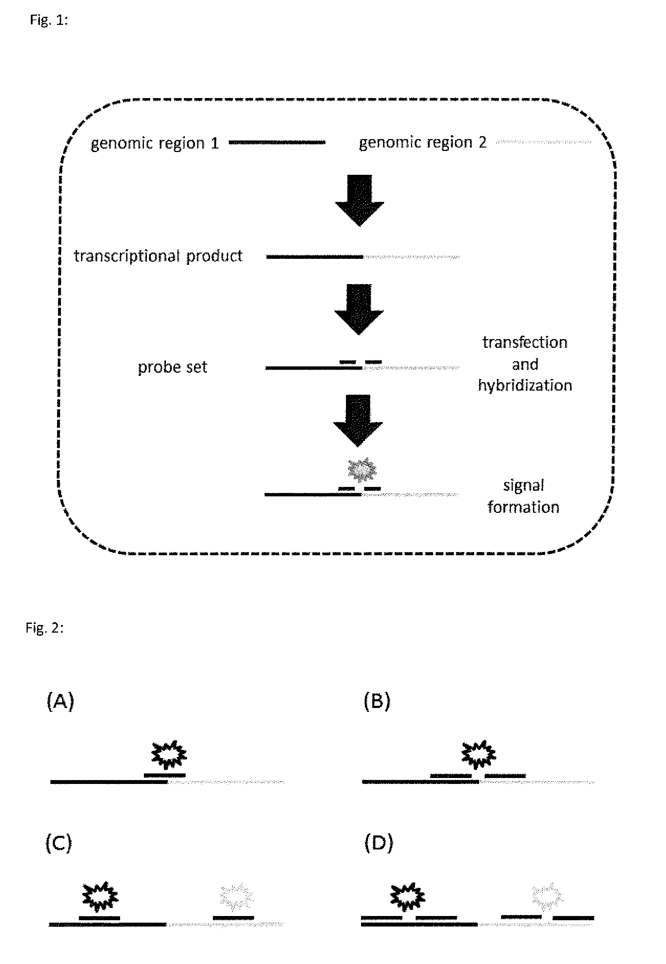

### Figure 1: Principle of the invention.

From a cell population, cells having a specific RNA (transcriptional product) in common are to be identified. This RNA may be formed by rearrangement or combination of genomic regions 1 and 2 and, thus, bears sequence information from genomic regions 1 and 2. The cells within the sample are transfected with a probe set which is specific to genomic regions 1 and 2. As an example, the probe set may comprise two probes. Upon hybridization of the complete probe set (e.g. 2 probes) to the RNA of interest, a signal may be detected which differs from cases where part (e.g. one probe) or none of the probe set is hybridized to the RNA.

### Figure 2: Preferred probe sets.

(A) A single probe spans the joint sequence from two different genomic regions. (B) A pair of probes interacting with each other spans the joint sequence stemming from two different genomic regions. (C) Two probes hybridize to different genomic regions without interacting with each other. (D) Two pairs of interacting probes hybridize to different genomic regions. The pairs do not interact with each other.

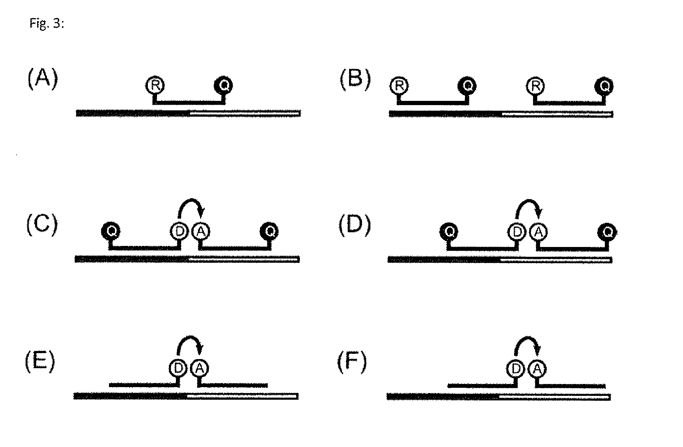

### Figure 3: Preferred combinations of single- and dual-labeled probes.

Figure 3 shows that single- or dual-labeled probes or combinations thereof may be used. A label can be a simple reporter R or acceptor A or donor D or a quencher Q. In some cases two different labels shall be used for determining whether one probe or both probes have been hybridized to the target. If two probes hybridize in close proximity to each other, FRET from donor to acceptor dyes (indicated by arrows) can occur (e.g. (C)-(F)). In some cases, one probe can span two genomic regions (e.g. (A), (D), (F)). Additional combinations are possible if more genomic regions are targeted with multiple probe sets.

### EXPERIMENTS

### 1) Enrichment of known murine hybridoma cells by CDR probes and FACSorting. Subsequent confirmation of enrichment by real-time qPCR enrichment analysis and confirmation of correct clonality by (Sanger) sequencing of amplified cDNA

Three different IgM hybridoma clones (5E4, KT13, and KT22) with known variable sequences are used for this experiment. The hybridoma cell line 5E4/1F1 was obtained from Prof. Vladka 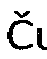 urin 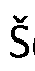 erbec at the Blood Transfusion Centre of Slovenia in Ljubljana, and the hybridoma cell lines KT13 and KT22 developed by Kazumasa Takeda / Asako Sugimoto (Takeda et al. 2008) were obtained from the Developmental Studies Hybridoma Bank developed under the auspices of the NICHD and maintained by The University of Iowa, Department of Biology, Iowa City, IA 15 52242.

Known sequences of the hybridoma VH and VL regions:

Cells in suspension culture in 75 cm² bottles are grown at 0.2-1 million cells/ml in DMEM medium (with 4.5g/l glucose, Gibco #41966-052) with 15% FBS (Biochrom #50615), 1x Penicillin/Streptomycin (Gibco #15140-122) and 1x GlutaMAX (Gibco #35050-038) at 37°C in 5% CO2 and 95% relative humidity. The KT22 clones' known variable sequences are chosen as hybridization targets. The KT22 clone-specific probe sets are designed to bind specifically in the regions of greatest respective variability of both VL and VH mRNAs. A dual molecular beacon-based probe set is chosen that allows greatest accessibility of the structured target mRNA with the following sequences:
Probes for KT22 VL domain
   KT22VLDonor (23nt, 2'-O-Me RNA) (SEQ ID NO. 7)
   5'-6FAM-GCCAUGCAAGUCAGGGCAUUGGC-BHQ1-3'
   KT22VLAcceptor (24nt, 2'-O-Me RNA) (SEQ ID NO. 8)
   5'-BHQ2-GAUGCGGGAGACACAGUCAGCAUC-5ROX-3'
Probes for KT22 VH domain
   KT22VHDonor (25nt, 2'-O-Me RNA) (SEQ ID NO. 9)
   5'-6FAM-GCACCUACUAUCCAGACAGUGGUGC-BHQ1-3'
   KT22VHAcceptor (25nt, 2'-O-Me RNA) (SEQ ID NO. 10)
   5'-BHQ2-ACCACUCGCAUCCAUUAGUAGUGGU-5ROX-3'

For demonstration of the efficiency of the cell sorting strategy, 5E4, KT13 and KT22 hybridoma cells are mixed in a specific ratio (e.g. 1:1:1) and used as starting cell population. The cells are treated according to Chen *et al.* using a toxin-based membrane permeabilisation. Briefly, streptolysin O (SLO) is activated using the reducing agent TCEP (Tris[2-carboxyethyl]phosphine hydrochloride). The activated SLO and molecular beacon (MB) sets in different combinations are then added to the cells for 10 min. Afterwards, the permeabilised cells are resealed by diluting the SLO mixture with fresh medium. Following a ∼1 h incubation period, the cells are washed and sorted by FACS according to King *et al.:* Fluorescence from each beacon is examined, and assayed for FRET by flow cytometry (Fig. 3). Emission at 520 nm is seen in the majority of donor beacon-transfected cells excited at 488 nm, whereas emission at 610 nm is observed in the majority of acceptor beacon-transfected cells excited at 560 nm. Only cells transfected with both donor and acceptor beacons show emission at both 520 nm and 610 nm when excited at 488 nm, establishing that FRET occurs between the 2 MB sets and is sufficient to allow cell sorting.

Cells sorted by FRET are finally analysed for presence and enrichment of both KT22-specific mRNAs (VH and VL) to confirm successful clonal enrichment. The mRNAs from the starting cell population and from the sorted cell population are extracted and compared by reverse transcription and enrichment analysis using real-time quantitative PCR (qPCR) with primer pairs for reverse transcription and amplification of partial sequences from 5E4, KT13 and KT22 mRNAs, and with primers for amplification of a control mRNA that is expressed at comparable levels across all cells under investigation (for example primer for amplification of a partial sequence from the Hspa5 mRNA used here). RT-qPCR is performed using the Verso SYBR Green 1-Step QRT-PCR kit (Thermo Scientific catalog no. AB-4104) according to the manufacturer's recommendations, comparing equal amounts of RNA extracted from the starting cell population and from the sorted cell population.
Primer for 5E4 VL (product size 136 bp):

| | |
|---|---|
| 5E4_VL_fwd (SEQ ID NO. 11) | 5'-cccagaaaatcggttggaaactt-3' |
| 5E4_VL_rev (SEQ ID NO. 12) | 5'-tcaagcctccatctcttgcagatc-3' |

Primer for KT13 VL (product size 123 bp):

| | |
|---|---|
| KT13_VL_fwd (SEQ ID NO. 13) | 5'-ccagaagccaagttggatgtgc-3' |
| KT13_VL_rev (SEQ ID NO. 14) | 5'-aaaggtcaccatgacctgcagg-3' |

Primer for KT22 VL (product size 120 bp):

| | |
|---|---|
| KT22_VL_fwd (SEQ ID NO. 15) | 5'-ctccatcttccaagttggttccat-3' |
| KT22_VL_rev (SEQ ID NO. 16) | 5'-cagtcagcatcacttgccatgc-3' |

Primer for 5E4 VH (product size 105 bp):

| | |
|---|---|
| 5E4_VH_fwd (SEQ ID NO. 17) | 5'-agatggtgaacctccctttcaca-3' |
| 5E4_VH_rev (SEQ ID NO. 18) | 5'-gccagtctccagagaagggactt-3' |

Primer for KT13 VH (product size 107 bp):

| | |
|---|---|
| KT13_VH_fwd (SEQ ID NO. 19) | 5'-tcaatgtggccttgtccttgaa-3' |
| KT13_VH_rev (SEQ ID NO. 20) | 5'-ctgggtaaaacagaggcctgga-3' |

Primer for KT22 VH (product size 102 bp):

| | |
|---|---|
| KT22_VH_fwd (SEQ ID NO. 21) | 5'-gaatcggcccttcacactgtct-3' |
| KT22_VH_rev (SEQ ID NO. 22) | 5'-atgtcttgggttcgccagactc-3' |

Control primer for Hspa5 (product size 156 bp):

| | |
|---|---|
| Hspa5_fwd (SEQ ID NO. 23) | 5'-aaaccaagacatttgccccaga-3' |
| Hspa5_rev (SEQ ID NO. 24) | 5'-cagcatctttggttgcttgtcg-3' |

Successful enrichment of KT22 cells from the hybridoma cell mixture by FRET-based cell sorting is demonstrated by relative enrichment of KT22-specific mRNA in comparison to 5E4- and KT13-specific mRNAs according to real-time qPCR results, using the ddCt analysis method with normalization to the Ct values from the amplification of the control mRNA Hspa5. The RT-PCR-amplified DNAs are separated on agarose gel, extracted and sequenced by standard Sanger capillary sequencing to confirm the correct identities.

### 2) Enrichment of a known human T cell clone in a diverse population by CDR probes and FACSorting. Subsequent confirmation of enrichment by real-time qPCR enrichment analysis and confirmation of correct clonality by (Sanger) sequencing of amplified cDNA

The human JM/Jurkat cell line is used as a T cell clone with known alpha and beta T cell receptor mRNA sequences (see NCBI nucleotide accession numbers X02592/M12959 for TCR alpha and K02885/X01417 for TCR beta). Jurkat cells (clone E6-1, positive for CD4 expression) are obtained from the American Type Culture Collection (ATCC number TIB-152) and cultured in RPMI-1640 medium (ATCC catalog no. 30-2001) supplemented with fetal bovine serum to a final concentration of 10% at 37°C in 5% CO2 and 95% relative humidity at densities between 0.1-1 million cells/ml.

A dual molecular beacon-based probe set is designed that specifically targets each CDR3 of both mRNAs with the following sequences:
Jurkat TCR alpha Probes:
   Donor (24nt, 2'-O-Me RNA) (SEQ ID NO. 25)
   5'-6FAM-GAG AUC ACU CAC AGC ACA GAU CUC-BHQ1
   Acceptor (24nt, 2'-O-Me RNA) (SEQ ID NO. 26)
   5'-BHQ2-CGA ACU UGG AAG CAC UGC UGU UCG-5ROX
Jurkat TCR beta Probes:
   Donor (23nt, 2'-O-Me RNA) (SEQ ID NO. 27)
   5'-6FAM-CAG GUC GAG AAA CUG CUG GCC UG-BHQ1
   Acceptor (26nt, 2'-O-Me RNA) (SEQ ID NO. 28)
   5'-BHQ2-GCU AAC AAG GUG UAG CCA UAG UUA GC-5ROX

The Jurkat cells are mixed with human CD4-positive T cells originating from a donor in various ratios (for example 1:1 and 1:10). Human CD4-positive T cells are isolated from whole blood using a suitable cell isolation kit, such as the RosetteSep Human CD4⁺ T Cell Enrichment Cocktail for immunodensity isolation of untouched CD4+ T cells (Stemcell Technologies catalog no. 15022) according to the manufacturer's recommendations. The mixed cell population is treated according to Chen *et al.* using a toxin-based membrane permeabilisation. Briefly, streptolysin O (SLO) is activated using the reducing agent TCEP (Tris[2-carboxyethyl]phosphine hydrochloride). The activated SLO and molecular beacon (MB) sets in different combinations are then added to the cells for 10 min. Afterwards, the permeabilised cells are resealed by diluting the SLO mixture with fresh medium. Following a ∼1 h incubation period, the cells are washed and sorted by FACS according to King *et al.:* Fluorescence from each beacon is examined, and assayed for FRET by flow cytometry (Fig. 3). Emission at 520 nm is again seen in the majority of donor beacon-transfected cells excited at 488 nm, whereas emission at 610 nm is observed in the majority of acceptor beacon-transfected cells excited at 560 nm. Only cells transfected with both donor and acceptor beacons show emission at both 520 nm and 610 nm when excited at 488 nm, establishing that FRET occurs between the 2 MB sets and is sufficient to allow cell sorting.

Cells sorted by FRET are finally analysed for presence and enrichment of both Jurkat-specific mRNAs (TCR alpha and TCR beta) to confirm successful clonal enrichment. The mRNAs from the starting cell population and from the sorted cell population are extracted and compared by reverse transcription and enrichment analysis using real-time quantitative RT-PCR (RT-qPCR) with primer pairs for reverse transcription and amplification of partial sequences from Jurkat TCR alpha and TCR beta mRNAs, and with primers for amplification of a control mRNA that is expressed at comparable levels across all cells under investigation (for example primer for amplification of a partial sequence from the HSPA5 mRNA used here). RT-qPCR is performed using the Verso SYBR Green 1-Step QRT-PCR kit (Thermo Scientific catalog no. AB-4104) according to the manufacturer's recommendations, comparing equal amounts of RNA extracted from the starting cell population and from the sorted cell population.
Primer for Jurkat TCR alpha (product size 145 bp):

| | |
|---|---|
| Jurkat_TCRA_fwd (SEQ ID NO. 29) | 5'-GAAACCTCCTTCCACCTGACGA-3' |
| Jurkat_TCRA_rev (SEQ ID NO. 30) | 5'-TATTTGGCCGGATGCTGAGTCT-3' |

Primer for Jurkat TCR beta (product size 149 bp):

| | |
|---|---|
| Jurkat_TCRB_fwd (SEQ ID NO. 31) | 5'-TGAAGATCCAGCCCTCAGAACC-3' |
| Jurkat_TCRB_rev (SEQ ID NO. 32) | 5'-CTCGGGTGGGAACACCTTGT-3' |

Control primer for HSPA5 (product size 149 bp):

| | |
|---|---|
| HSPA5_fwd (SEQ ID NO. 33) | 5'-CACAGTGGTGCCTACCAAGAAGTC-3' |
| HSPAS_rev (SEQ ID NO. 34) | 5'-CAGGAGGAATTCCAGTCAGATCAAA-3' |

Successful enrichment of Jurkat cells from the T cell mixture by FRET-based cell sorting is demonstrated by relative enrichment of Jurkat-specific mRNAs according to real-time qPCR results, using the ddCt analysis method with normalization to the Ct values from the amplification of the control mRNA HSPA5. The RT-PCR-amplified DNAs are separated on agarose gel, extracted and sequenced by standard Sanger capillary sequencing to confirm the correct identities.

## Claims

1. A method of clonally sorting and isolating living immune cells, which have a specific ribonucleic acid molecule in common, wherein the ribonucleic acid molecule is the transcriptional product of at least two different genomic regions, the method comprising the steps of:
a. providing a sample comprising said living cells;
b. providing at least one labeled probe set, each probe set comprising
i. a first probe which is specific for a region of the ribonucleic acid stemming from a first genomic region and,
ii. a second probe which is specific for a region of the ribonucleic acid stemming from a second genomic region and,
c. transfecting said cells with said probe set such that hybridization to said regions of the ribonucleic acid molecule may occur and if said hybridization occurs the probes lie adjacent to one another on said ribonucleic acid molecule;
d. detecting hybridization of said probe set to said nucleic acid regions; and
e. sorting the cells depending on how hybridization to said regions has occurred,
wherein the immune cells are hybridoma cells and/or B cells and
wherein at least two genomic regions encode for different complementary determining regions of the light chain or heavy chain of an antibody.

2. The method according to claim 1, wherein the first probe is a single-labeled probe and the second probe is a single-labeled probe, and wherein one label consists of a reporter molecule and the other label consists of a quencher molecule.

3. The method according to claim 1, wherein the first probe is a dual-labeled probe and the second probe is a dual-labeled probe, and wherein each dual-label consists of a reporter molecule and a quencher molecule.

4. The method according to claim 1, wherein the first probe is a dual-labeled probe and the second probe is a single-labeled probe, and wherein the single-label consists of a quencher molecule.

5. The method according to one of the preceding claims, wherein a second probe set is provided, and wherein the second probe set comprises a third probe which is specific for a region of the ribonucleic acid stemming from a third genomic region and a fourth probe which is specific for a region of the ribonucleic acid stemming from a fourth genomic region.

6. The method according to any of the preceding claims, wherein the reporter is selected from the group of fluorescein dyes and the quencher is selected from the group of rhodamine dyes.

7. The method according to one of the claims 3 to 6, wherein the dual-labeled probe is a Molecular Beacon probe.

8. The method according to any of the preceding claims, wherein the step of detecting hybridization is based on quenching or fluorescence resonance energy transfer (FRET).

9. The method according to any of the preceding claims, wherein the step of sorting the cells is based on fluorescence activated cell sorting (FACS).

10. The method according to any of the preceding claims, wherein the ribonucleic acid molecule encodes for one subunit of an antibody

## Patentansprüche

1. Verfahren zur klonalen Sortierung und Isolierung von lebenden Immunzellen, die gemeinsam ein spezifisches Ribonukleinsäuremolekül haben, wobei das Ribonukleinsäuremolekül ein Transkriptionsprodukt von mindestens zwei verschiedenen genomischen Regionen ist, das Verfahren umfassend die Schritte:
a. bereitstellen einer Probe umfassend die lebenden Zellen;
b. bereitstellen von mindestens einem markierten Sondensatzes, jeder Sondensatz beinhaltend
i. eine erste Sonde, die spezifisch für einen Bereich der aus einer ersten genomischen Region stammenden Ribonukleinsäure ist und,
ii. eine zweite Sonde, die spezifisch für einen Bereich der aus einer zweiten genomischen Region stammenden Ribonukleinsäure ist und
c. transfizieren der Zellen mit den Sondensätzen, so dass Hybridisierung auf die Regionen der Ribonukleinsäuremoleküle stadtfinden kann und wenn die Hybridisierung stadtfindet die Sonden neben einander auf dem Ribonukleinsäuremolekül liegen;
d. nachweisen der Hybridisierung des Sondensatzes auf den Nukleinsäurebereichen; und
e. sortieren der Zellen abhängig davon wie die Hybridisierung zu den Bereichen stattgefunden hat,
wobei die Immunzellen Hybridomazellen und/oder B-Zellen sind und
wobei mindestens zwei genomische Bereiche für verschiedene Complementary Determining Regions der leichten Kette oder der schweren Kette eines Antikörpers kodieren

2. Verfahren nach Anspruch 1, wobei die erste Sonde eine einfachmarkierte Sonde ist und die zweite Sonde eine einfachmarkierte Sonde ist, und wobei eine Markierung aus einem Reportermolekül besteht und die andere Markierung aus einem Quenchermolekül besteht.

3. Verfahren nach Anspruch 1, wobei die erste Sonde eine doppelmarkierte Sonde ist und die zweite Sonde eine doppelmarkierte Sonde ist, und wobei jede Doppelmarkierung aus einem Reportermolekül und einem Quenchermolekül besteht.

4. Verfahren nach Anspruch 1, wobei die erste Sonde eine doppelmarkierte Sonde ist und die zweite Sonde eine einfachmarkierte Sonde ist, und wobei die Einfachmarkierung aus einem Quenchermolekül besteht.

5. Verfahren nach einem der vorhergehenden Ansprüche, wobei ein zweiter Sondensatz bereitgestellt wird, und wobei der zweite Sondensatz eine dritte Sonde, die spezifisch für einen Bereich des von einem dritten genomischen Bereich stammenden Ribonükleinsäuremolekuls ist und eine vierte Sonde, die spezifisch für einen Bereich des von einem dritten genomischen Bereich stammenden Ribonükleinsäuremolekuls ist beinhaltet.

6. Verfahren nach einem der vorhergehenden Ansprüche, wobei der Reporter aus der Gruppe der Fluoreszein-Farbstoffe und der Quencher aus der Gruppe der Rhodamin-Farbstoffe ausgewählt ist.

7. Verfahren nach einem der Ansprüche 3 bis 6, wobei die doppelmarkierte Sonde eine Molecular Beacon Sonde ist.

8. Verfahren nach einem der vorhergehenden Ansprüche, wobei der Detektionsschritt der Hybridisierung auf Quenching oder Fluorescence Resonance Energy Trasfer (FRET) basiert.

9. Verfahren nach einem der vorhergehenden Ansprüche, wobei der Sortierungsschritt der Zellen auf Fluorescence Activated Cell Sorting (FACS) basiert.

10. Verfahren nach einem der vorhergehenden Ansprüche, wobei das Ribonukleinsäuremolekül für eine Untereinheit eines Antikörpers kodiert.

## Revendications

1. Procédé pour trier clonalement et isoler des cellules immunitaires vivantes qui ont une molécule d'acide ribonucléique spécifique en commun, où la molécule d'acide ribonucléique est le produit de la transcription d'au moins deux régions génomiques différentes, le procédé comprenant les étapes :
a. fournir un échantillon comprenant lesdites cellules vivantes ;
b. fournir au moins un jeu de sondes marquées, chaque jeu de sonde comprenant
i. une première sonde spécifique pour une région de l'acide ribonucléique issu d'une première région génomique et,
ii. une second sonde spécifique pour une région de l'acide ribonucléique issu d'une seconde région génomique et,
c. transfecter lesdites cellules avec ledit jeu de sondes de manière à ce que l'hybridation auxdites régions de la molécule d'acide ribonucléique soit possible et de manière à ce que si ladite hybridation se produit les sondes soient adjacentes l'une par rapport à l'autre sur ladite molécule d'acide ribonucléique ;
d. détecter l'hybridation dudit jeu de sondes à ladite région d'acide nucléique ; et
e. trier les cellules selon comment l'hybridation auxdites régions s'est produite,
où les cellules immunitaires sont des hybridomes et/ou des lymphocytes B et
où au moins deux régions génomiques codent pour des régions déterminantes de complémentarité différentes de la chaîne légère ou lourde d'un anticorps.

2. Le procédé selon la revendication 1, où la première sonde est une sonde à marquage unique et la seconde sonde est une sonde à marquage unique, et où un marquage consiste en une molécule rapporteur et l'autre marquage consiste en une molécule quencher.

3. Le procédé selon la revendication 1, où la première sonde est une sonde à double marquage et la deuxième sonde est une sonde à double marquage, et où chaque double marquage consiste en une molécule rapporteur et une molécule quencher.

4. Le procédé selon la revendication 1, où la première sonde est une sonde à double marquage et la second sonde est une sonde à marquage unique, et où le marquage unique consiste en une molécule quencher.

5. Le procédé selon l'une quelconque des revendications précédentes, où un second jeu de sondes est fourni, et où le second jeu de sondes comprend une troisième sonde qui est spécifique pour une région de l'acide ribonucléique issu d'une troisième région génomique et une quatrième sonde qui est spécifique pour une région de l'acide ribonucléique issu d'une quatrième région génomique.

6. Le procédé selon l'une quelconque des revendications précédentes, où le rapporteur est sélectionné du groupe des colorants fluorescéines et le quencher est sélectionné du groupe des colorants rhodamines.

7. Le procédé selon l'une quelconque des revendications 3 à 6, où la sonde à double marquage est une sonde Molecular Beacon.

8. Le procédé selon l'une quelconque des revendications précédentes, où l'étape de détection de l'hybridation est basée sur le transfert d'énergie par résonance de quenching ou le transfert d'énergie par résonance de fluorescence (FRET).

9. Le procédé selon l'une quelconque des revendications précédentes, où l'étape de tri des cellules est basée sur le tri cellulaire par cytofluorométrie (FACS).

10. Le procédé selon l'une quelconque des revendications précédentes, où la molécule d'acide ribonucléique code pour une sous-unité d'un anticorps.
